# EUROPEAN PATENT SPECIFICATION

(11) **EP 1 740 189 B1**
(45) Date of publication and mention of the grant of the patent: **13.06.2012**
(21) Application number: 05718468.1
(22) Date of filing: 18.04.2005
(51) Int. Cl.: A61K 9/12, A61K 9/00, A61K 31/66, A61K 31/683, A61K 31/685

(54) **LUNG SURFACTANT SUPPLEMENTS**
LUNGEN-SURFACTANT-ERGÄNZUNGSMITTEL
COMPLEMENTS DE SURFACTANT PULMONAIRE

(30) Priority: 19.04.2004 US 563690 P
(43) Date of publication of application: 10.01.2007
(73) Proprietor: CENTRE NATIONAL DE LA RECHERCHE SCIENTIFIQUE (CNRS), 75016 Paris (FR); Universite Louis Pasteur Industrie, F-67000 Strasbourg (FR)
(72) Inventor: KRAFFT, Marie-Pierre, F-67000 Strasbourg (FR); VANDAMME, Thierry, F-67200 Strasbourg-Cronenbourg (FR); GERBER, Frédéric, F-68620 Bitschwiller (FR); SHIBATA, Osamu, Fukuoka 814-0013 (JP)
(74) Representative: Colombet, Alain André
(86) International application number: PCT/IB2005/001020
(87) International publication number: WO 2005/099718

(56) References cited:
- WO-A-01/85136
- WO-A-03/061593
- WO-A-2004/071488
- WO-A-2005/044226
- US-A- 5 576 016
- US-A1- 2001 046 474
- US-A1- 2002 037 316
- US-B1- 6 630 169
- WOLFSON M R ET AL: "Pulmonary applications of perfluorochemical liquids: Ventilation and beyond" PAEDIATRIC RESPIRATORY REVIEWS, W.B. SAUNDERS, vol. 6, no. 2, June 2005 (2005-06), pages 117-127, XP004903109 ISSN: 1526-0542
- COURRIER H M ET AL: "In vivo evaluation of a reverse water-in-fluorocarbon emulsion stabilized with a semifluorinated amphiphile as a drug delivery system through the pulmonary route" INTERNATIONAL JOURNAL OF PHARMACEUTICS, AMSTERDAM, NL, vol. 282, no. 1-2, 10 September 2004 (2004-09-10), pages 131-140, XP004543675 ISSN: 0378-5173

## Description

### FIELD OF THE INVENTION

The present invention relates generally to lung surfactant compositions and methods for treating pulmonary diseases. The invention specifically discloses the use of biocompatible highly fluorinated compounds, including fluorocarbons in the treatment of various pulmonary conditions.

### BACKGROUND OF THE INVENTION

The native lung surfactant consists of a complex mixture of phospholipids, neutral lipids and proteins. The surfactant comprises roughly 90% lipids and 10% proteins with a lipid composition of 55% dipalmitoylphosphatidylcholine (DPPC), 25% phosphatidylcholine (PC), 12% phosphatidylglycerol (PG), 3.5% phosphatidylethanolamine (PE), sphingomyelin and phosphatidylinositol (PI).

The lung surfactant functions are to reduce the surface tension within the alveoli. It helps to mediate the transfer of oxygen and carbon dioxide, promotes alveolar expansion and covers the surface of the lung alveoli. Reduced surface tension permits the alveoli to be held open under low pressure. In addition, the lung surfactant supports alveolar expansion by varying the surface tension depending on alveolar size.

Surfactant supplements are presently used therapeutically when the lung surfactant present does not allow efficient respiratory function. Surfactant supplementation is commonly used to treat Respiratory Distress Syndrome (RDS), when surfactant deficiencies compromise pulmonary function. While RDS is primarily a disease of newborn infants, an adult form of the disease, Adult Respiratory Distress Syndrome (ARDS), has many characteristics in common with RDS, thus lending itself to similar therapies. More generally, there exist various forms of acute lung injuries that are related to a dysfunction of the pulmonary surfactant.

The primary etiology of RDS is attributed to insufficient amounts of pulmonary surfactant. Those at greatest risk are infants born before the 36th week of gestation having premature lung development. Neonates born at less than 28 weeks of gestation have a 60-80% chance of developing RDS. RDS is a life-threatening condition.

Adult respiratory distress occurs as a complication of numerous conditions, including shock-inducing trauma, infection, burn or direct lung damage. The pathology is observed histologically as diffuse damage to the alveolar wall, with hyaline membrane formation and capillary damage. Hyaline membrane formation, whether in ARDS or RDS, creates a barrier to gas exchange. Decreased oxygen delivery produces a loss of lung epithelium, resulting in decreased surfactant production and foci of collapsed alveoli. This can initiate a vicious cycle of hypoxia and lung damage.

In those pathologies wherein the native lung surfactant is deficient or not functional, molecules of DPPC are immobilized in the form of crystalline islets and cannot re-spread onto the alveolar surface, which can greatly impair the respiratory cycle.

Surfactant replacement therapy has recently been used either alone or in combination with ventilation therapy. Early work with surfactant replacements used preparations of human lung surfactant obtained from lung lavage.

The second generation of surfactant substitutes are purified preparations of bovine and porcine lung surfactant. Like human surfactant, bovine lung surfactant preparation is complex. Sources are few and availability is limited. Further, while the use of bovine lung surfactant in neonates does not present a problem immunologically, bovine surfactant applications in adults could immunologically sensitize patients to other bovine products. Moreover, the absence of risk of transmission of the Creutzfeld-Jakob disease remains to be proven.

Formulations of synthetic surfactants have also been proposed. For instance, EP 050 793 discloses a combination of hexadecanol and DPPC.

Ventilation with liquid fluorocarbons has been proposed in U.S patent No. 5,853,003, and was shown to be safe, improve lung function and recruit lung volume in patients with severe respiratory distress (Greenspan et al., Biomedical Instrumentation and Technology (1999) 33:253-259 ; Leach et al., New England Journal of Medicine (1996) 335:761-767). The biocompatibility of fluorocarbons has been discussed (Riess, Chemical Reviews (2001) 101:2797-2919; Riess, Tetrahedron (2002) 58:4113-4131).

### SUMMARY OF THE INVENTION

A Langmuir DPPC monolayer is accepted as a pulmonary surfactant model. Upon compression, a DPPC monolayer undergoes a phase transition from a fluid liquid-expended state to a crystalline liquid-condensed state. Crystalline islets are thus formed due to the high cohesive energy of the DPPC molecules. During expansion (in the course of a respiration cycle), the molecules of DPPC involved in crystalline islets do not respread rapidly enough on the alveoli surface. For that reason, DPPC alone cannot function as an effective lung surfactant.

The inventors have discovered that highly fluorinated compounds, and in particular fluorocarbons, in a liquid or gaseous form, make it possible to inhibit the liquid-expended to liquid-condensed transition, and therefore prevent the formation of crystalline islets when a Langmuir phospholipid monolayer is compressed.

On that basis, it is provided a method for improving the fluidity and/or spreadability of the native lung surfactant in a human or an animal in need of such treatment, wherein the human or animal is administered with a fluorocarbon composition.

The fluorocarbon composition may be in a liquid or gaseous form, and may be administered neat as an aerosol or vaporized.

A preferred highly fluorinated compound is a fluorocarbon such as perfluorooctylethane (PFOE) or bis(perfluorobutyl)ethene (F-44E). A still preferred fluorocarbon is a brominated fluorocarbon, and a still more preferred fluorocarbon is perfluorooctyl bromide (PFOB).

In a preferred embodiment, the fluorocarbon is administered in association with a phospholipid, such as dipalmitoylphosphatidylcholine (DPPC).

In the case where the fluorocarbon is used in association with a phospholipid, the molecular ratio between these two components preferably ranges from 1 to 300.

Only a small amount of fluorocarbon is necessary for improving the fluidity of the pulmonary surfactant monolayer.

The fluorocarbon is administered in liquid or vapor (gaseous) form, and preferably in a quantity corresponding of about 0.005 to about 4 % of fluorocarbon in volume/kg body weight, preferably from about 0.005 % to about 2 % in volume/kg body weight.

The fluorocarbon may be administered neat or in the form of a water-in-fluorocarbon emulsion, or in the form of an oil-in-fluorocarbon emulsion.

The fluorocarbon composition may also serve as a drug delivery system. It is then contemplated that a further therapeutic agent can be dispersed in the fluorocarbon preparation.

The invention further provides a therapeutic composition comprising a fluorocarbon and a phospholipid, and optionally a therapeutic agent, e.g. prednisone or epinephrine.

In a preferred embodiment, the composition is in the form of an emulsion comprising a continuous phase of fluorocarbon. In a further preferred embodiment, the emulsion with a continuous fluorocarbon phase contains pulmonary pharmacologically active substance such as an antibiotic, a tuberculostatic antimycobacterial agent, an anticancer agent, a pulmonary vasoactive substance, etc.

### LEGEND OF THE FIGURES

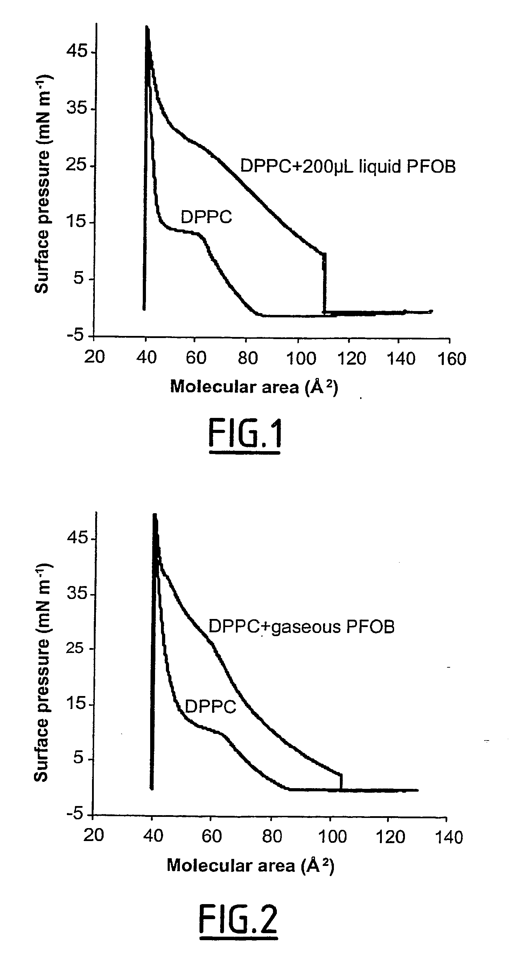

Figure 1 is a graph showing the variation of the surface pressure versus the molecular area, for DPPC alone and for DPPC contacted with 200 µl of liquid PFOB. The graph shows the influence of liquid PFOB on the DPPC monolayer. The plateau reflecting the coexistence of the fluid phase and the crystalline islets, clearly visible on the isotherm of DPPC alone, is no longer seen on the isotherm of DPPC contacted with the liquid fluorocarbon.
Figure 2 is a graph showing the surface pressure versus the molecular area, for DPPC alone and for DPPC contacted with a nitrogen atmosphere saturated with gaseous PFOB. The graph shows the influence of gaseous PFOB on the DPPC monolayer. The plateau reflecting the coexistence of the fluid phase and the crystalline islets, clearly visible on the isotherm of DPPC alone, is no longer seen on the isotherm of DPPC contacted with the gaseous fluorocarbon.

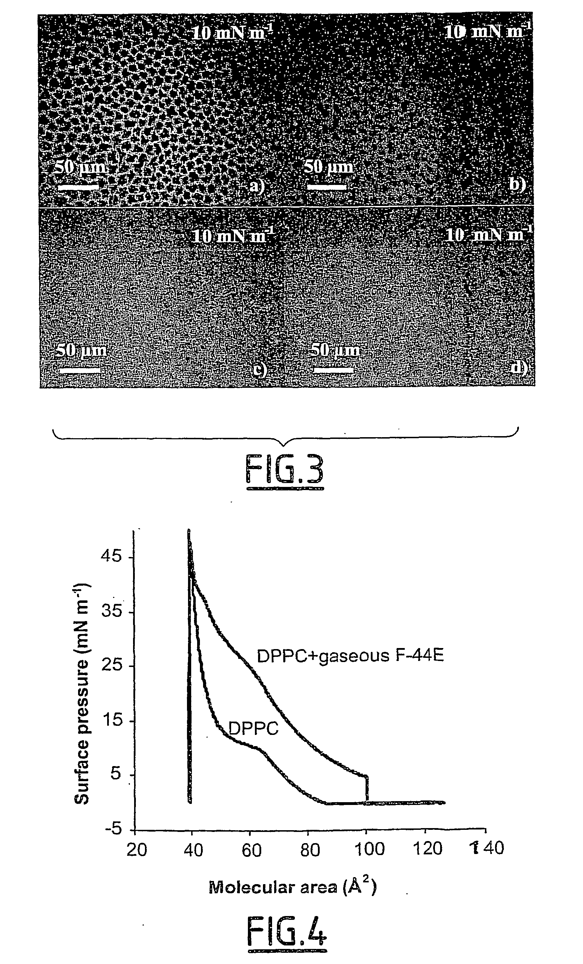

Figure 3 is a set of photographs by fluorescence microscopy showing that the crystalline islets, initially present in the DPPC monolayer compressed at 10 mN.m⁻¹ (A) progressively disappear with time when the monolayer is contacted with gaseous PFO B (B, C, D, visualization of the monolayer after 3, 5 and 7 min, respectively).
Figure 4 is a graph showing the surface pressure versus the molecular area, for DPPC alone and for DPPC contacted with a nitrogen atmosphere saturated with gaseous bis(perfluorobutyl)ethene (F-44E). The graph shows the influence of gaseous F-44E on the DPPC monolayer. The plateau reflecting the coexistence of the fluid phase and the crystalline islets, clearly visible on the isotherm of DPPC alone, is no longer seen on the isotherm of DPPC contacted with the gaseous F-44E.

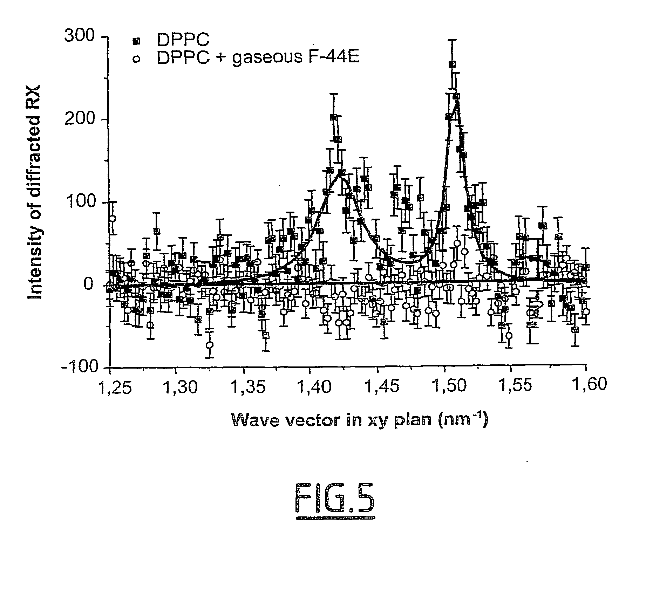

Figure 5 shows the analysis by grazing incidence X-ray diffraction of the DPPC monolayer compressed at 40 mN.m⁻¹ before (■) and after (○) the addition of gaseous F-44E. The two diffraction peaks seen in the absence of fluorocarbon are characteristics of the molecular organisation of DPPC in crystalline islets (■). After saturation with gaseous F-44E, one can clearly see that these peaks have disappeared (○), which shows the dissolution of the islets and the fluidization of the DPPC monolayer.

### DETAILED DESCRIPTION OF THE INVENTION

Compounds useful in this invention (hereinafter called "fluorocarbons") may be straight, branched or cyclic, saturated or unsaturated poly- or perfluorinated hydrocarbons. These compounds may also comprise heteroatoms such as oxygen or nitrogen, or halogen atoms other than fluorine, in particular bromine. Generally, the compound is liquid or gaseous at room temperature (25°C). Preferably, the compound has from 2 to 12 carbon atoms. There is a large number of compounds compatible with the biomedical application described.

One may use for example straight or branched chain fluorocarbons such as perfluoropropane, perfluorobutane, perfluorohexane, perfluorooctane, bis(*F-*alkyl)ethenes, CₙF₂ₙ₊₁CH=CHCₙ,F_{2n'-1}, such as iC₃F₉CH=CHC₆F₁₃ ("F-i36E"), C₄F₉CH=CHC₄F₉ ("*F*-44E"), C₆F₁₃CH=CHC₆F₁₃ ("F-66E"), cyclic fluorocarbons, such as C₁₀F₁₈ (*F*-decalin, perfluorodecalin or "FDC"), *F*-di- or *F-*trimethylbicyclo-3,3,1-nonane ("nonane"); perfluorinated amines, such as *F-*tripropylamine ("FTPA") and *F*-tributylamine ("FTBA"), *F*-4-methyloctahydroquinolizine ("FMOQ"), *F*-n-methyldecahydroisoquinoline ("FMIQ"), *F*-n-methyldecahydroquinoline ("FHQ"), *F*-n-cyclohexylpyrrolidine ("FCHP") and *F*-2-butyltetrahydrofuran ("FC-75" or "RM101"). Any other fluorocarbons contemplated for biomedical uses such as those described by Riess (Chemical Reviews (2001) 101:2797-2919) can be used.

Other fluorocarbons include brominated perfluorocarbons, such as 1-bromo-heptadecafluorooctane (C₈F₁₇Br, sometimes designated perfluorooctyl bromide or "PFOB"), 1-bromopentadecafluoroheptane (C₇F₁₅Br), and 1-bromotridecafluorohexane (C₆F₁₃Br, sometimes known as perfluorohexyl bromide or "PFHB"). Other brominated fluorocarbons are disclosed in U.S. Pat. No. 3,975,512. Also contemplated are fluorocarbons having other nonfluorine substituents, such as perfluorooctyl chloride, perfluorohexyl dichloride, perfluorooctyl hydride, and similar compounds having different numbers of carbon atoms.

Additional fluorocarbons contemplated in accordance with this invention include perfluoroalkylated ethers or polyethers, such as (CF₃)₂CFO(CF₂CF₂)₂OCF(CF₃)₂ ; (CF₃)₂CFO(CF₂CF₂)₃OCF(CF₃) ; (CF₃)CFO(CF₂CF₂)F ; (CF₃)₂CFO(CF₂CF₂)₂F ; (C₆F₁₃)₂O. Further, fluorocarbon-hydrocarbon compounds, such as, for example compounds having the general formula CₙF₂ₙ₊₁C_{n'}H_{2n'+1} (also called semifluorinated alkanes or -FnHn', CₙF₂ₙ₊₁OC_{n'}H_{2n'+1}; or CₙF₂ₙ₊₁CH=CHC_{n'}H_{2n'+1}, where n and n' are the same or different and n ranges from 4 to 10 and n' ranges from 2 to 20. Such compounds include, for example, perfluorooctylethane (C₈F₁₇C₂H₅, "PFOE"), perfluorohexylethane (C₆F₁₃C₂H₅, "PFH E") and perfluorohexyldecane (C₆F₁₃C₁₀H₂₁, "F6H10") or perfluorobutyl-1-undecene (C₄F₉CH=CHC₁₀H₂₁ "F4H8E"). It will be appreciated that esters, thioethers, and other variously modified mixed fluorocarbon-hydrocarbon compounds are also encompassed within the broad definition of "fluorocarbon" materials suitable for use in the present invention. Mixtures of fluorocarbons are also contemplated.

Preferred fluorocarbons are perfluorooctyl bromide (PFOB), bis(perfluorobutyl) ethene (F-44E) and perflu orooctylethane (PFOE).

In addition, the fluorocarbon may be neat or may be combined with other materials, such as surfactants (including fluorinated surfactants) and dispersed materials.

In particular, a therapeutic agent may be combined (*e.g*. dispersed) with the fluorocarbon.

Suitable therapeutic agents include, but are not limited to, antibiotics such as gentamicin, erythromycin a nd doxycycline; tuberculostatic antimycobacterials such as pyrazinamide, ethambutol and isoniazid; anticancerous agents such as cis-platinum, cyclophosphamide, 5-fluorouracyl and doxorubicin; pulmonary vasoactive substances and regulators of pulmonary hypertension such as tolazoline; respiratory stimulants such as doxapram; vasoactive bronchodilators or bronchostrictors such as acetylcholine, priscoline, epinephrine and theophylline; mucolytic agents such as acetylcysteine; steroids such as cortisone or prednisone; antiviral agents such as ribavirin.

There exist abundant data that demonstrate the biocompatibility of fluorocarbons. They are amenable to sterilization techniques. For example, they can generally be heat-sterilized (such as by autoclaving) or sterilized by radiation. In addition, sterilization by ultrafiltration is also contemplated.

The fluorocarbon can be provided as a liquid or in a gaseous form.

Inhalation or forced (positive pressure) introduction, either nasal or oral, of the fluorocarbon composition can be achieved by any of a variety of methods known in the art. These include mechanical suspension by agitation of the composition in a closed chamber followed by inhalation, or forced introduction of the suspension from an opening in the chamber. Microparticles can be inhaled from standard aerosol delivery systems that are well known in the art. The patient may receive a particulate suspension which is placed into an air stream such as by injection of the dispersed composition into a positive pressure ventilation tube or into an endotracheal tube at the moment of inspiration or when air is forced into the lungs. Metered dosages may be mechanically injected into such devices. The fluorocarbon composition may be dispersed in air by using the Venturi effect, where air is moved at right angles across a Venturi tube causing the fluorocarbon composition to be drawn through the tube and dispersed into the air that is inhaled or mechanically introduced into the lungs. Pulsatile delivery of the fluorocarbon composition in a volume of gas and inhalation of the aerosolized bolus is also known in the art as described in PCT published application WO 94/07514, and the delivery techniques described therein can be used in the present invention.

Liquid compositions of fluorocarbons may be directed to specific regions of the patient's pulmonary air passages by a number of different conventional means, such as a bronchoscope, a catheter, and the like.

The fluorocarbon introduced into the patient's lung may be in liquid or vapor form. The quantity of fluorocarbon composition administered may correspond to about 0.005 to about 4 % of liquid fluorocarbon in volume/kg body weight, preferably from about 0.005 % to about 2 %.

The method of the invention improves the fluidity of the pulmonary surfactant, mainly by preventing the formation of crystalline islets of phospholipid, such as crystalline islets of DPPC, and/or by respreading the phospholipid islets that might have formed.

This is useful in the context of pulmonary surfactant deficiency, as in the diseases listed below.

In addition to RDS in neonates, ARDS in adults caused by severe hypovolemic shock, lung contusion, diver's lung, post-traumatic respiratory distress, post-surgical atelectasis, septic shock, multiple organ failure, Mendelssohn's disease, obstructive lung disease, pneumonia, pulmonary oedema or any other condition resulting in lung surfactant deficiency or respiratory distress are all candidates for fluorocarbon supplementation and the method of the invention.

Treatment of pulmonary fibrosis, emphysema, and chronic bronchitis can all benefit from fluorocarbon therapy as proposed herein.

In a preferred embodiment, the fluorocarbon is administered with a surfactant agent, *e.g*. a lipid or a phospholi pid. The surfactant agent may be an amphiphilic molecule or a mixture of amphiphilic molecules, or may be a preparation of artificial lung surfactant, or may be extracted from human or mammal lung surfactant. Preferably, the su rfactant agent is a phospholipid. The phospholipid may be a mixture of phospholipids. It is preferably selected from the group consisting of native pulmonary surfactant phospholipids, *i.e.* dipalmitoylphosphatidylcholine (DPPC), distearoylphosphatidylcholine, phosphatidylcholine (PC), phosphatidylglycerol (PG), dioleophosphatidylethanolamine (PE), dipalmitoylphosphatidylamine, sphingomyelin and phosphatidylinositol (PI), cardiolipids, soja and egg yolk phospholipids. In another embodiment, the fluorocarbon may also be administered with a mixture of phospholipids and glycoproteins, such as SP-A, SP-B, SP-C and SP-D, which are components of the native pulmonary surfactant composition. Fluorocarbon compounds are generally poorly soluble in water. Therefore, it is possible to prepare emulsions comprising a fluorocarbon compound, a phospholipid and water.

Such emulsions allow easy administration of both one or more fluorocarbons and one or more phospholipids at controlled dosage.

The emulsions may be prepared by mixing an aqueous phase and, dissolved or dispersed in the water, the phospholipid, with the fluorocarbon phase, optionally in presence of an additional suitable surfactant. One or both phases may further contain one or more therapeutic agents, as described above.

Appropriate surfactants include in particular fluorinated amphiphilic compounds such as fluorinated dimorpholinophosphate.

Preferably, the water phase is added slowly to the fluorinated phase containing an emulsifier, in particular a fluorinated surfactant.

The resulting pre-emulsion may then be homogenized, using procedures that are well known as such, in order to yield an emulsion with a narrow droplet size distribution.

In the case where the fluorocarbon is used in association with a phospholipid, the fluorocarbon/phospholipid molecular ratio between these two components preferably ranges from1 to 300. In a preferred embodiment, the molecular ratio between phospholipid and the fluorocarbon ranges from1 to 50.

An example of composition useful in the invention is a therapeutic composition comprising an emulsion of water in PFOB that contains DPPC. Another example of composition is a therapeutic composition comprising an emulsion of water in PFOE that contains DPPC.

Further examples of compositions include these above compositions supplemented with a pulmonary active agent, such as prednisone or epinephrine.

The invention thus contemplates the use of a fluorocarbon, optionally in combination with a surfactant agent, especially a phospholipid, e.g. a phospholipid of a native pulmonary surfactant, for the manufacture of a medicament intended for improving the spreadability and/or the fluidity of the native pulmonary surfactant in patients in need of such treatment.

The patients may be a human or a non-human animal, preferably a mammal. It may be a new-born patient, an infant, a child or an adult of any age.

The invention will be further illustrated by the following examples.

### EXAMPLES

The monolayer of native pulmonary surfactant was modeled by a Langmuir monolayer of DPPC. In this model, the DPPC molecules were spread using a spreading solvent onto the surface of water contained in a trough coated with Teflon^{®}. After evaporation of the solvent, the DPPC molecules were progressively compressed using two mobile Teflon^{®} barriers. The variation of the surface pressure was measured as a function of the compression by a pressure sensor such as a Wilhelmy blade.

### Example 1:

### Fluidization of a monolayer of dipalmito ylphosphatidylcholine (DPPC) with liquid perfluorooctyl bromide (PFOB)

Small quantities of perfluorooctyl bromide (PFO B) in liquid form (1-200 µl), contacted with the DPPC monolayer, result in the disappearance of the liquid expanded / liquid condensed phase transition of the phospholipid. Thus, in presence of PFOB, the DPPC molecules did not form crystalline islets and respread easily on the water surface. Figure 1 compares the compression isotherms of a DPPC monolayer alone and in contact with the fluorocarbon. In absence of fluorocarbon, one notes the presence of a plateau reflecting the coexistence of the expanded liquid state (fluid phase) and the condensed liquid state (crystalline islets). This plateau disappeared totally in presence of fluorocarbon. The DPPC monolayer is totally fluid.

### Example 2 :

### Fluidization of a DPPC monolayer by gaseous PFOB

Placing the Langmuir trough in a close space saturated with PFOB vapour, it is possible to study the interactions between the DPPC monolayer and the gaseous PFOB. As shown in figure 2, the plateau that reflects the coexistence of the expanded liquid state (fluid phase) and the condensed liquid state (crystalline islets) disappears when the monolayer is contacted with the gaseous PFOB.

The DPPC monolayer is totally fluid.

Fluorescence microscopy makes it possible to visualize the dissolution of crystalline islets of DPPC molecules when the monolayer is contacted with gaseous PFOB (Figure 3A). After 3 minutes, the size of the islets becomes small (Figure 3B) and after 5 minutes, the phenomenon is becoming accurate: the DPPC monolayer has almost completely re-spread (Figure 3C) and after 7 minutes, the islets have completely disappeared (Figure 3D).

### Example 3 :

### Fluidization of a DPPC monolayer by gaseous bis(perfluorobutyl)ethene (F-44E)

Similarly, when the DPPC monolayer is contacted with gaseous F-44E, the plateau reflecting the coexistence of the expanded liquid state and the condensed liquid state disappears, as shown in the compression isotherm of DPPC (Figure 4).

A grazing incidence X-ray diffraction study of the effect of gaseous F-44E on the DPPC monolayer clearly evidenced the disappearance of the crystalline islets and efficient re-spreading of the molecules of DPPC in the presence of gaseous F-44E (Figure 5).

### Example 4 :

### Vaporization of PFOB

2.50 ml of sterile PFOB were placed in the tank of vaporiser/pneumatic compressor such as the Omron CX3 aerosol, optionally fitted with a vaporisation kit for Omron CX3, or any other vaporisation system (Hudson TUp-draftll/Pulmo-aide, Airlife Mysty/Pulmo-Aide, Respirgard/Pulmo-aide, etc). PFOB was then vaporized according to the recommendations of the manufacturer.

### Example 5 :

### Aerosolisation of PFOB using HFA-227 as propellent gas

6 ml of PFOB were introduced in an aluminium receptacle (Cebal, Clichy, France) (23.6 x 60 x 20 mm ; 20 ml) (ST004, GL001 a nd TD 00033, Lablabo, Annemasse, France) that can contain a volume of 1 ml.

Using an appropriate filling system (Pamasol P20 16, Switzerland), 4 ml of 1,1,1,2,3,3,3-heptafluoropropane (HFA-227, Solkane® 227 pharma) were introduced into the aluminium receptacle. The receptacle was closed with a jet and a button.

### Example 6 :

### Aerosolisation of PFOB with N₂ as propellent gas

The protocole of example 5 was followed by replacing HFA-227 by N₂ and using an appropriate filling system.

### Example 7 :

### Preparation of an emulsion of physiological water in PFOB

An emulsion containing 5% v/v physiological water (NaCl solution 0.9 % w/v), 3% w/v perfluorooctyl (undecyl)dimorpholinophosphate (F8H11DMP, used as the emulsifying agent) and 95% v/v PFOB was prepared. F8H11DMP (3 g) was dispersed in 95 ml of PFOB using a low energy mixer (Ultra-Turrax® T25 equipped with the S25N25F, Ika-Labortechnik, Stanfen, Germany) at a temperature below 40° C. 5 ml of physiological water were then added dropwise under constant agitation.

The pre-emulsion thus obtained was homogenized under high pressure using a Microfluidizer^{®} 110T (Microfluidics, New Jersey, USA) for about 10 minutes at a temperature below 40° C. The emulsion thus obtained was opalescent, and the mean size of the water droplets was 60 ± 5 nm (3000 Zeta Sizer, Malvern Instrument). This emulsion was sterilized by filtration through a 0.22 µm membrane before bottling in 10 ml flasks (with 8% hold-up volume). The mean size of the water droplets after one-year storage at 25°C was 100 ± 7 nm.

### Example 8 :

### Preparation of an emulsion of physiological water in PFOB containing DPPC

An emulsion containing 5% v/v water, 5% w/v DPPC, 3% w/v perfluorooctyl (undecyl)dimorpholinophosphate and 95% v/v PFOB, was prepared. The perfluorooctyl(undecyl)dimorpholinophosphate was dispersed in the PFOB using a low energy mixer (Ultra-Turrax) at a temperature below 40° C, as described in Example 7. Separately, a dispersion of DPPC (5% w/v) in physiological water was prepared using an Ultra-Turrax mixer. The aqueous phospholipid dispersion (5 ml) was then added dropwise to the fluorinated phase, while maintaining the agitation.

The pre-emulsion thus obtained was homogenized under high pressure using a Microfluidizer^{®} 110T (Microfluidics, New Jersey, USA) for about 10 minutes at a temperature below 40°C as described in Example 1. The emulsion thus obtained was opalescent, and the mean size of the water droplets was 70 ± 5 nm (3000 Zeta Sizer, Malvern Instrument). This emulsion was sterilized, bottled and stored as described in Example 7. The mean size of droplets after one-year storage at 25°C was 120 ± 10 nm.

### Example 9 :

### Preparation of an emulsion of physiological water in perfluorooctylethane (PFOE) containing DPPC

The emulsion is prepared following the protocol of Example 8, using PFOE instead of PFOB. An opalescent emulsion is obtained. The mean size of the water droplets was 65 ± 5 nm (3000 Zeta Sizer, Malvern Instrument). The emulsion was sterilized, bottled and stored as described in Example 7. The mean size of the droplets after one-year storage at 25° C was 110 ± 10 nm.

### Example 10 :

### Preparation of an emulsion of isotonic buffer in PFOB containing DPPC and a vasoactive bronchodilatator agent, epinephrine

An emulsion containing 5 % v/v isotonic buffer (pH 3,0 - 3.5), 0.02 w/v epinephrine, 1 % w/v DPPC, 3 % w/v F8H11DMP (perfluorooctyl)undecyl dimorpholinophosphate) and 95 % v/v PFOB is obtained as follows : 0.75 g F8H11DMP were dispersed in 23.75 ml of PFOB as described in Example 7. Separately, 0.25 g DPPC and 5 mg epinephrine were co-dispersed in 1.25 ml of isotonic buffer. This dispersion was added dropwise to the fluorinated phase under constant agitation. The pre-emulsion obtained was homogenised under high pressure with an Emulsiflex-B3^{®} (Avestin, Ottawa, Canada). An opalescent emulsion was obtained, with water droplets of a mean size of 40 ± 3 nm (3000 Zeta Sizer, Malvern Instrument). This emulsion was sterilized, bottled and stored as described in Example 7. The mean size of the droplets after one-year storage at 25°C was 70 ± 5 nm.

### Example 11 :

### Preparation of an emulsion of physiological water in PFOE containing DPPC and epinephrine

The emulsion was prepared following the protocol of Example 10, using PFOE instead of PFOB. An opalescent emulsion was obtained with droplets of a mean size of 55 ± 5 nm (3000 Zeta Sizer, Malvern Instrument). The emulsion was sterilized, bottled and stored as described in Example 7. The mean size of the droplets after one year storage at 25°C was 118 ± 10 nm.

### Example 12 :

### Preparation of an emulsion of physiological water in PFOB containing an anti-inflammatory agent, prednisone

An emulsion containing 4.7 % v/v physiological water, 0.02 w/v prednisone, 20 % v/v perfluorobutyl-1-undecene (F4H8E), 4.7 % v/v perfluorohexyldecane (F6H10), 4.8 w/v perfluorooctyl(undecyl)dimorpholinophosphate (F8H11DMP) and 67 % v/v PFOB is obtained as follows : 2 mg prednisone are dissolved in 2 ml of F4H8E under agitation and moderate heating. 0.5 ml F6H10 and 7 ml PFOB (containing 0.5 g of F8H11DMP) were then subsequently added at room temperature. 0.5 ml of physiological water were then added to the solution under agitation using an Ultra-Turrax^{®} T25. The pre-emulsion is then homogenised under high pressure using an Emulsiflex-B3^{®}. An opalescent emulsion was obtained, with droplets of a mean size of 1 25 ± 12 nm (3000 Zeta Sizer, Malvern Instrument). This emulsion was sterilized by membrane filtration and stored in vessels of 2.5 ml, as described in Example 7. The mean size of the droplets after six-month storage at 25°C was 170 ± 20 nm.

### Example 13 :

### Preparation of an emulsion of physiological water in PFOE containing prednisone

The emulsion was prepared following the protocol of Example 12, using PFOE instead of PFOB. An opalescent emulsion is obtained with droplets of a mean size of 146 ± 15 nm (3000 Zeta Sizer, Malvern Instrument). The emulsion was sterilized, bottled and stored as described in Example 12. The mean size of the water droplets after six-months storage at 25°C was 210 ± 25 nm.

### Example 14 :

### Vaporisation of an emulsion of physiological water in PFOB

The emulsion prepared according to Example 7 was vaporised following the protocol described in Example 4.

### Example 15 :

### Aerosolization of an emulsion of physiological water in PFOB containing DPPC (propellent gas : HFA-227)

The emulsion prepared according to Example 8 was aerosolized following the protocol described in Example 5.

### Example 16 :

### Aerosolization of an emulsion of physiological water in PFOB containing DPPC and epinephrine (propellent gas : HFA-227)

The emulsion prepared according to Example 10 was aerosolized following the protocol described in Example 5.

### Example 17 :

### Aerosolization of an emulsion of physiological water in PFOB containing prednisone (propellent gas : HFA-227)

The emulsion prepared according to Example 12 is aerosolized following the protocol described in Example 5.

## Claims

1. A fluorocarbon composition for use as a medicament for improving the fluidity and/or the spreadability of the native lung surfactant in a human or animal.

2. The fluorocarbon composition for use according to claim 1, wherein the fluorocarbon composition is in the form of an aerosol.

3. The fluorocarbon composition for use according to claim 1, wherein the fluorocarbon composition is in a vapor form.

4. The fluorocarbon composition for use according to claim 1, wherein the fluorocarbon composition contains, or is associated with a surfactant agent.

5. The fluorocarbon composition for use according to claim 4, wherein the surfactant agent is a lipid.

6. The fluorocarbon composition for use according to claim 4, wherein the surfactant agent is a phospholipid.

7. The fluorocarbon composition for use according to claim 6, wherein the phospholipid is dipalmitoylphosphatidylcholine (DPPC).

8. The fluorocarbon composition for use according to claim 1, wherein the fluorocarbon composition is suitable to be administered in a liquid or vapor form in a quantity corresponding to about 0.005 to about 4 % of liquid fluorocarbon in volume per kg body weight.

9. The fluorocarbon composition for use according to claim 1, wherein the fluorocarbon composition is suitable to be administered in the form of a water-in-fluorocarbon emulsion.

10. The fluorocarbon composition for use according to claim 1, wherein the fluorocarbon is suitable to be administered in the form of an oil-in-fluorocarbon emulsion.

11. The fluorocarbon composition for use according to claim 1, wherein a further therapeutic agent is dispersed in the fluorocarbon composition.

12. The fluorocarbon composition for use according to claim 1, wherein the fluorocarbon is perfluorooctyl bromide (PFOB).

13. The fluorocarbon composition for use according to claim 1, wherein the fluorocarbon is bis(perfluobutyl)ethene (F-44E).

14. The fluorocarbon composition for use according to claim 1, wherein the fluorocarbon is a semifluorinated alkane of formula CₙF₂ₙ₊₁C_{n'}H₂ₙ₊₁, with n ranging from 4 to 10 and n' ranging from 2 to 20.

15. The fluorocarbon composition for use according to claim 1, wherein the fluorocarbon is perfluorooctylethane (PFOE).

16. A therapeutic composition comprising a fluorocarbon and a phospholipid that is in a form of an emulsion comprising a continuous phase of fluorocarbon.

17. The composition of claim 16, wherein the fluorocarbon is perfluorooctyl bromide (PFOB).

18. The composition of claim 16, wherein the fluorocarbon is bis(perfluorobutyl)ethene (F-44E).

19. The composition of claim 16, wherein the fluorocarbon is a semifluorinated alkane of formula CₙF₂ₙ₊₁C_{n'}H_{2n'+1}, with n ranging from 4 to 10 and n' ranging from 2 to 20.

20. The composition of claim 16, wherein the fluorocarbon is perfluorooctylethane (PFOE).

21. The composition of claim 16, wherein the phospholipid is dipalmitoylphosphatidylcholine (DPPC).

22. The composition of claim 16, further comprising a therapeutic agent.

23. The composition of claim 16, comprising a water-in-PFOB emulsion, that contains DPPC and prednisone or epinephrine.

## Patentansprüche

1. Fluorkohlenstoff-Zusammensetzung zur Verwendung als Medikament für die Verbesserung der Fluidität und/oder Streichfähigkeit von nativem Lungensurfactant in einem Menschen oder Tier.

2. Fluorkohlenstoff-Zusammensetzung zur Verwendung gemäß Anspruch 1, wobei die Fluorkohlenstoff-Zusammensetzung in der Form eines Aerosols vorliegt.

3. Fluorkohlenstoff-Zusammensetzung zur Verwendung gemäß Anspruch 1, wobei die Fluorkohlenstoff-Zusammensetzung in Dampfform vorliegt.

4. Fluorkohlenstoff-Zusammensetzung zur Verwendung gemäß Anspruch 1, wobei die Fluorkohlenstoff-Zusammensetzung ein Tensidsystem enthält oder damit verbunden ist.

5. Fluorkohlenstotf-Zusammensetzung zur Verwendung gemäß Anspruch 4, wobei das Tensidsystem ein Lipid ist.

6. Fluorkohlenstoff-Zusammensetzung zur Verwendung gemäß Anspruch 4, wobei das Tensidsystem ein Phospholipid ist.

7. Fluorkohlenstoff-Zusammensetzung zur Verwendung gemäß Anspruch 6, wobei das Phospholipid Dipatmitoylphosphatidylcholin (DPPC) ist.

8. Fluorkohlenstoff-Zusammensttzung zur Verwendung gemäß Anspruch 1, wobei die Fluorkohlenstoff-Zusammensetzung geeignet ist in flüssiger oder dampfförmiger Form in einer Menge, die etwa 0,005 bis etwa 4 % in Volumen pro kg Körpergewicht entspricht, verabreicht zu werden.

9. Flüorkohlenstoff-Zusammensetzung zur Verwendung gemäß Anspruch 1, wobei die Fluorkohlenstoff-Zusammensetzung geeignet ist in der Form einer Wasser-in-Fluorkohlenstoff Emulsion verabreicht zu werden.

10. Fluorkohlenstoff-Zusammensetzung zur Verwendung gemäß Anspruch 1, wobei der Fluorkohlenstoff geeignet ist in Form einer Öl-in-Fluorkohlenstoff Emulsion verabreicht zu werden.

11. Fluorkohlenstoff-Zusammensetzung zur Verwendung gemäß Anspruch 1, wobei ein weiterer therapeutischer Wirkstoff in der Fluorkohlenstoff-Zusammensetzung dispergiert ist.

12. Fluorkohlenstaff-Zusammensetzung zur Verwendung gemäß Anspruch 1, wobei der Fluorkohlenstoff Perfluoroctylbromid (PFOB) ist.

13. Fluorkohlenstoff-Zusammensetzung zur Verwendung gemäß Anspruch 1, wobei der Fluorkohlenstoff Bis(perfluorbutyl)ethen (F-44E) ist.

14. Fluorkohlenstoff-Zusammensetzung zur Verwendung gemäß Anspruch 1, wobei der Fluorkohlenstoff ein semifluoriniertes Alkan der Formel CₙF₂ₙ₊₁C_{n'}H2ₙ₊₁ ist, mit n im Bereich von 4 bis 10 und n' im Bereich von 2 bis 20.

15. Fluorocarbon-Zusammensetzung zur Verwendung gemäß Anspruch 1, wobei der Fluorkohlenstoff Perfluoroctylethan (PFOE) ist.

16. Therapeutische Zusammensetzung enthaltend einen Fluorkohlenstoff und ein Phospholipid, welches in Form einer Emulsion vorliegt, die eine kontinuierliche Phase an Fluorkohlenwasserstoff enthält.

17. Zusammensetzung gemäß Anspruch 16, wobei der Fluorkohlenwasserstoff Perfluoroctylbromid (PFOB) ist.

18. Zusammensetzung gemäß Anspruch 16, wobei der Fluorkohlenwasserstoff Bis(perfluorbutyl)ethen (F-44E) ist.

19. Zusammensetzung gemäß Anspruch 16, wobei der Fluorkohlenwasserstoff ein semifluoriniertes Alkan der Formel CₙF₂ₙ₊₁C_{n'}H_{2n'+1} ist, mit n im Bereich von 4 bis 10 und n' im Bereich von 2 bis 20.

20. Zusammensetzung gemäß Anspruch 16, wobei der Fluorkohlenwasserstoff Perfluoroctylethan (PFOE) ist.

21. Zusammensetzung gemäß Anspruch 16, wobei das Phospholipid Dipamitoylphosphatidycholin (DPPC) ist.

22. Zusammensetzung gemäß Anspruch 16, welche ferner einen therapeutischen Wirkstoff enthält.

23. Zusammensetzung gemäß Anspruch 16, weiche eine Wasser-in-PFOB Emulsion enthält, die DPPC und Prednison oder Epinephrin aufweist.

## Revendications

1. Composition fluorocarbonée destinée à être utilisés comme médicament pour améliorer la fluidité et/ou la capacité de dispersion du tensioactif pulmonaire natif chez un humain ou un animal.

2. Composition fluorocarbonée destinée à être utilisée selon la revendication 1, dans laquelle la composition fluorocarbonée a la forme d'un aérosol.

3. Composition fluorocarbonée destinée à être utilisée selon la revendication 1, dans laquelle la composition fluorocarbonée a la forte d'une vapeur.

4. Composition fluorocarbonée destinée à être utilisée selon la revendication 1, dans laquelle la composition fluorocarbonée contient un agent tensioactif ou est associée à un tel agent.

5. Composition fluorocarbonée destinée à être utilisée selon la revendication 4, dans laquelle l'agent tensioactif est un lipide.

6. Composition fluorocarbonée destinée être utilisée selon la revendication 4, dans laquelle l'agent tensioactif est un phospholipide.

7. Composition fluorocarbonée destinée à être utilisée selon la revendication 6, dans laquelle ie phospholipide est dipalmitoylphosphatidylcholine (DPPC).

8. Composition fluorocarbonée destinée à être utilisée selon la revendication 1, dans laquelle la compositions fluorocarbonée est adaptée pour être administrée sous une forme de liquide ou de vapeur dans une quantité correspondant à environ 0,005 jusqu'à environ 4 % de fluorocarbone liquide en volume par kilo de poids corporel.

9. Composition fluorocarbonée destinée à être utilisée selon la revendication 1, dans laquelle la compositions fluorocarbonée est adaptée pour être administrée sous la forme d'une émulsion de fluorocarbone dans de l'eau.

10. Composition fluorocarbonée destinée à être utilisée selon la revendication 1, dans laquelle le fluorocarbone est adapté pour être administré sous la forme d'une émulsion de fluorocarhone dans de l'huile.

11. Composition fluorocarbonée destinée à être utilisée selon la revendication 1, dans laquelle un agent thérapeutique supplémentaire est dispersé dans la composition fluorocarbonée.

12. Composition fluorocarbonée destinée à être utilisée selon la revendication 1, dans laquelle le fluorocarbone est du bromure de perfluorooctyle (PFOB)

13. Composition fluorocarbonée destinée à être utilisée selon la revendication 1, dans laquelle le fluorocarbone est bis(perfluobutyl)éthène (F-44E).

14. Composition fluorocarbonée destinée à être utilisée selon la revendication 1, dans laquelle le fluorocarbone est un alcane semi fluoré de formule CₙF₂ₙ₊₁CₙH_{2n'+1}, n étant dans la plage de 4 à 10 et n' dans la plage de 2 à 20.

15. Composition fluorocaroonée destinée à être utilisée selon la revendication 1, dans laquelle le fluorocarbone est du perfluorooctyléthane (PFOE).

16. Composition thérapeutique comprenant un fluorocarbone et un phospholipide qui est sous forme d'une émission comprenant une phase continue de fluorocarbone.

17. Composition selon la revendication 16, dans laquelle le fluorocarbone est du bromure de perfluorooctyle (PFOB)

18. Composition selon la revendication 16, dans laquelle le fluorocarbone est bis(perfluobutyl)éthéne (F-44E).

19. Composition, selon la revendication 16, dans laquelle le fluorocarbone est un alcane semi fluoré de formule CₙF₂ₙ₊₁C_{n'}H_{2n'+1}. n étant dans la plage de 4 à 10 et n' dans la plage de 2 à 20.

20. Composition selon la revendication 16, dans laquelle le fluorocarbone est perfluorooctyléthane (PFOE).

21. Composition selon la revendication 16, dans laquelle le phospholipide est dipalmitoylphosphatidylcholine (DPPC).

22. Composition selon la revendication 16, comprenant de plus un agent thérapeutique.

23. Composition selon la revendication 16, comprenant une émulsion de PFOB dans de l'eau, qui contient DPPC et prednisone ou épinéphrine,
